# EUROPÄISCHE PATENTSCHRIFT

(11) **EP 3 880 056 B1**
(45) Veröffentlichungstag und Bekanntmachung des Hinweises auf die Patenterteilung: **04.09.2024**
(21) Anmeldenummer: 19816206.7
(22) Anmeldetag: 14.11.2019
(51) Int. Cl.: A61B 1/00, A61B 34/00, H01H 3/14, H01H 21/26, A61C 1/00, G06F 3/01, A61B 17/00

(54) **VERFAHREN UND VORRICHTUNG ZUR AUSFÜHRUNG VERSCHIEDENER FUNKTIONEN IM ZUSAMMENHANG MIT CHIRURGISCHEN GERÄTEN**
METHOD AND DEVICE FOR CARRYING OUT VARIOUS FUNCTIONS IN CONNECTION WITH SURGICAL INSTRUMENTS
PROCÉDÉ ET APPAREIL POUR EXÉCUTER DIFFÉRENTES FONCTIONS ASSOCIÉES À DES APPAREILS CHIRURGICAUX

(30) Priorität: 16.11.2018 DE 102018128848
(43) Veröffentlichungstag der Anmeldung: 22.09.2021
(73) Patentinhaber: Olympus Winter & Ibe GmbH, 22045 Hamburg (DE)
(72) Erfinder: HOLTKÖTTER, Jannis, 20357 Hamburg (DE)
(74) Vertreter: Hoener, Matthias
(86) Internationale Anmeldenummer: PCT/EP2019/081299
(87) Internationale Veröffentlichungsnummer: WO 2020/099545

(56) Entgegenhaltungen:
- DE-A1- 10 336 276
- DE-A1- 10 336 276
- DE-A1- 102014 207 127
- DE-U1- 20 001 134
- DE-U1- 20 001 134
- US-A1- 2005 128 184
- US-A1- 2005 128 184
- US-A1- 2006 116 667
- US-A1- 2006 116 667
- US-A1- 2009 021 476
- US-A1- 2009 021 476
- US-A1- 2011 106 068
- US-A1- 2011 106 068

## Beschreibung

Die Erfindung betrifft eine Vorrichtung zur Ausführung verschiedener Funktionen im Zusammenhang mit chirurgischen Geräten gemäß dem Oberbegriff des Anspruchs 1. Eine Vorrichtung gemäß dem Oberbegriff des unabhängigen Anspruchs 1 ist aus der Druckschrift US 2011/106068 A1 bekannt. Des Weiteren betrifft die Erfindung ein Verfahren zur Betätigung eines chirurgischen Gerätes gemäß Anspruch 12.

Chirurgische Geräte der hier genannten Art dienen als Instrumente zur Durchführung chirurgischer Untersuchungen oder Operationen am menschlichen Körper. Beispielsweise können derartige, vorzugsweise als Handinstrumente ausgebildete, Geräte dazu verwendet werden, Gegenstände zu halten oder zu entfernen. Des Weiteren können die Geräte dazu ausgebildet sein, insbesondere Gewebe, zu schneiden oder sonstige Funktionen während einer Behandlung bzw. Operation zu übernehmen, wie das Ausleuchten einer Körperhöhle, die Aufnahme von Fotos oder Videos oder das Durchspülen mit einer Flüssigkeit. Bei diesen chirurgischen Geräten kann es sich beispielsweise um Endoskope, Resektoskope, Ultraschallgeneratoren, Spülpumpen, Kamerasysteme, Lichtquellen, Wiederaufbereitungsmaschinen oder dergleichen handeln. Ein Großteil dieser Geräte wird manuell bedient bzw. gesteuert. Dazu weisen die betreffenden Geräte entsprechende Vorrichtungen bzw. Handgriffe auf. Darüber hinaus ist es bekannt, dass einige Funktionen der genannten chirurgischen Geräte über einen Fußschalter durch eine Bedienperson bzw. einen Operateur ausführbar sind. Durch einen Fußschalter lässt sich der manuelle Aktionsspielraum des Operateurs erweitern. Fußschalter kommen bekanntermaßen bei Geräten zum Einsatz, die sich auch über den Fuß bedienen bzw. aktivieren lassen. Bei diesen Funktionen handelt es sich oftmals um einfache An- und Ausschaltfunktionen. Als Beispiel hierfür kann die Bedienung eines HF- oder Ultraschallgenerators, aber auch einer Spülpumpe, einer Lichtquelle, einer Wiederaufbereitungsmaschine oder dergleichen genannt werden.

Bekannte Fußschalter weisen mindestens ein, vorzugsweise zwei Pedale, bzw. Fußpedale, auf, welche durch den Fuß des Operateurs betätigbar sind. Bei zwei oder mehr Pedalen des Fußschalters kann jedem Pedal eine gesonderte Funktion zugesprochen werden. Damit die Pedalen besser unterscheidbar sind, können diese beispielsweise farblich markiert sein. Neben den Pedalen können die Fußschalter weitere Bedienelemente, wie beispielsweise einen Knopf, aufweisen, durch den sich die Möglichkeiten der Fußbedienung noch erweitern lassen.

Damit die Fußpedalen während der Operation nicht versehentlich betätigt werden, kann um die Schalter herum ein seitlicher Trittschutz angeordnet sein. Für den Fall, dass ein Fußschalter zwei oder mehr Pedalen aufweist, können die einzelnen Pedalen außerdem untereinander durch einen erhöhten Steg voneinander getrennt werden. So kann sichergestellt werden, dass immer nur ein Pedal zurzeit durch den Operateur betätigbar wird und nicht versehentlich auf zwei Fußpedale gleichzeitig getreten wird.

Zur Kommunikation bzw. zur Energieversorgung können die Fußschalter mit den chirurgischen Geräten über ein Kabel verbunden sein. Gleichermaßen ist es denkbar, dass die Schalter über eine kabellose Verbindung mit den chirurgischen Geräten kommunizieren. Die Energieversorgung der Fußschalter erfolgt sodann durch Batterien bzw. Akkus.

Es ist durchaus üblich, dass während einer Behandlung bzw. während einer Operation mehrere chirurgische Geräte über jeweils einen Fußschalter bedient werden. Dazu werden vor der Behandlung bzw. vor der Operation die einzelnen Fußschalter auf dem Fußboden des Behandlungsraumes aufgebaut und mit den entsprechenden Geräten gekoppelt. Insbesondere bei der Verwendung einer Vielzahl von Fußschaltern kann dies für die Bedienperson bzw. den Operateur sehr unübersichtlich werden. Die Gefahr einer Fehlbedienung der Fußschalter steigt. Darüber hinaus ist der zur Verfügung stehende Raum für die Platzierung der Fußschalter in dem Behandlungsraum limitiert.

Daraus ergibt sich für die vorliegende Erfindung die Aufgabe, ein Verfahren sowie eine Vorrichtung zu schaffen, mit der verschiedene Funktionen im Zusammenhang mit chirurgischen Geräten auf eine einfache und sichere Art und Weise ausführbar sind.

Eine Vorrichtung zur Lösung dieser Aufgabe weist die Merkmale des Anspruchs 1 auf. Demnach ist es vorgesehen, dass einem Fußschalter einer Vorrichtung zur Ausführung verschiedener Funktionen im Zusammenhang mit einem chirurgischen Gerät mindestens ein Projektor zugeordnet ist, durch den mindestens ein virtuelles Bedienfeld erzeugbar ist. Dieses mindestens eine virtuelle Bedienfeld kann neben dem Fußschalter auf einen Fußboden des Behandlungsraumes projiziert werden. Bei diesem projizierten Bedienfeld kann es sich um ein Symbol für einen Schalter handeln oder um eine Projektion eines Befehls wie beispielsweise *Start* oder *Stopp* oder *Licht an*/*aus.* Des Weiteren ist es erfindungsgemäß vorgesehen, dass der Fußschalter einen Sensor zur Erfassung des virtuellen Bedienfeldes aufweist. Bei diesem Sensor kann es sich um einen CCD-Chip oder einen sonstigen Sensor zur Wahrnehmung von wenigstens sich ändernden Kontrasten handeln. Über diesen mindestens einen Sensor lassen sich beispielsweise Veränderungen des virtuellen Bedienfeldes wahrnehmen und für die Ausführung der entsprechenden Funktion verarbeiten. Bei dem durch den Projektor erzeugten Bedienfeld handelt es sich um ein virtuelles Bedienfeld, da dieses nur sichtbar ist, wenn es durch den Projektor auf den Fußboden projiziert wird. Durch diesen mindestens einen Projektor und diesen mindestens einen Sensor lässt sich jeder Fußschalter durch mindestens ein weiteres Bedienfelder erweitern, und zwar ohne, dass weitere Fußschalter auf dem Fußboden des Behandlungsraumes zu installieren sind.

Insbesondere kann es die vorliegende Erfindung weiter vorsehen, dass der den mindestens einen Projektor und den mindestens einen Sensor aufweisende Fußschalter mit mindestens einem chirurgischen Gerät, insbesondere einem Endoskop, einem Resektoskop, einem HF-Resektoskop, einem Kamerasystem, einen HF- oder Ultraschallgenerator, einer Spülpumpe, einer Lichtquelle, einer Wiederaufbereitungsmaschine oder dergleichen, über ein Kabel oder kabellos koppelbar ist. Demnach kann es vorgesehen sein, dass der Projektor und/oder der Sensor über Funk oder Bluetooth mit dem entsprechenden chirurgischen Gerät oder eine weitere Steuereinheit verbunden ist. Für die kabellose Kopplung zwischen dem Projektor und dem Sensor kann es vorgesehen sein, dass dem jeweiligen chirurgischen Gerät ein entsprechender Receiver zum Austausch von Informationen zuordbar ist. Bei einem kabelgebundenen Austausch von Informationen zwischen dem Projektor und dem Sensor und dem entsprechenden chirurgischen Gerät kann ein entsprechendes Kabel zum Informations- aber auch Energietransport entweder direkt an den Fußschalter oder das chirurgische Gerät gekoppelt werden. Auf diese Art und Weise entsteht eine sichere und zuverlässige Kopplung zwischen den genannten Komponenten.

Bevorzugterweise kann es des Weiteren vorgesehen sein, dass das virtuelle Bedienfeld für die Ausführung verschiedener Funktionen im Zusammenhang mit den chirurgischen Geräten konfigurierbar, insbesondere programmierbar ist. So ist das virtuelle Bedienfeld nicht eingeschränkt auf die Ausführung einer einzigen Funktion im Zusammenhang mit einem oder mehreren der genannten chirurgischen Geräte. Vielmehr kann das virtuelle Bedienfeld in Abhängigkeit von der durchzuführenden Funktion umprogrammiert werden, sodass es beispielsweise für aufeinanderfolgende Behandlungen bzw. Operationen verschiedene Funktionen initiieren kann.

Außerdem kann das virtuelle Bedienfeld durch unterschiedliche Betätigung, beispielsweise eine kurze oder eine lange Betätigung durch einen Fuß, verschiedene Funktionen ausführen. Auf diese Art und Weise lässt sich die erfindungsgemäße Vorrichtung auf eine besonders flexible Art und Weise nutzen.

Ein weiteres besonders bevorzugtes Ausführungsbeispiel der vorliegenden Erfindung kann es vorsehen, dass über den mindestens einen Sensor eine Betätigung des virtuellen Bedienfeldes durch eine Bedienperson, insbesondere durch einen Fuß oder eine Geste der Bedienperson, feststellbar ist, woraufhin durch die festgestellte Betätigung die entsprechende Funktion im Zusammenhang mit dem an den Fußschalter gekoppelten chirurgischen Gerät initiierbar ist. So ist es denkbar, dass nicht nur die bloße Betätigung des virtuellen Bedienfeldes durch einen Fuß, beispielsweise durch Abdunkelung des Bedienfeldes, eine Funktion initiierbar ist, sondern auch durch eine Geste. Bei dieser Geste kann es sich beispielsweise um ein Führen des Fußes, aber auch einer Hand, entlang des virtuellen Bedienfeldes handeln. So lässt sich beispielsweise durch eine Geste die Helligkeit einer Lichtquelle, dem Dimmer-Prinzip entsprechend, nahezu kontinuierlich ändern.

Weiter kann es bevorzugt vorgesehen sein, dass der mindestens eine Projektor und der mindestens eine Sensor an dem mindestens ein Fußpedal aufweisenden Fußschalter anbaubar sind. Es ist denkbar, dass ein oder mehrere Projektoren und Sensoren den bekannten Fußschaltern zuordbar sind, indem sie beispielsweise an selbige anbaubar sind. Auf diese Art und Weise lassen sich die bekannten Fußschalter auf nahezu beliebige Art und Weise durch die hier beschriebene Vorrichtung erweitern. Gleichermaßen ist es jedoch auch denkbar, dass der Projektor und der Sensor eine eigene Einheit bilden und zusammen mit den bekannten Fußschaltern auf dem Fußboden platziert werden.

Schließlich kann es weiter vorgesehen sein, dass es sich bei dem Projektor um einen Laserprojektor handelt. Durch die Erzeugung eines virtuellen Bedienfeldes mittels Lasertechnologie lassen sich besonders vielseitige und darüber hinaus verschiedenfarbige Bedienfelder erzeugen, welche durch die Bedienperson auf eine intuitive und verständliche Art und Weise nutzbar sind. Es kann vorgesehen sein, dass der mindestens eine Projektor und der mindestens eine Sensor zur Versorgung mit elektrischer Energie eine Batterie oder einen Akku aufweisen. Gleichermaßen ist es denkbar, dass der mindestens eine Projektor und der mindestens eine Sensor zur Energieversorgung mit den Fußpedalen kabelgebunden sind.

Eine weitere vorteilhafte Weiterbildung der vorliegenden Erfindung kann es vorsehen, dass über das virtuelle Bedienfeld Funktionen ohne Sicherheits- und/oder Gesundheitsrisiken ausführbar sind, wie beispielsweise eine Aufnahme eines Screenshots oder dergleichen. So lässt sich das Risiko einer fehlerhaften Anwendung des virtuellen Bedienfeldes vermeiden. Es kann darüber hinaus vorgesehen sein, dass für kritische Funktionen, die durch das virtuelle Bedienfeld ausführbar sind, zunächst eine Betätigung eines Fußpedals des Fußschalters oder eines anderen Bedienelementes notwendig ist. So lässt sich die Funktion über das virtuelle Bedienfeld erst initiieren, sofern vorab ein anderes Bedienelement entsprechend betätigt wurde. Auf diese Art und Weise die Handhabung verschiedener Bedienelemente zur Ausführung einer Funktion zu kombinieren, kann der Aktionsraum der Bedienperson auf eine Vielzahl von Funktionen erweitert werden.

Bevorzugt ist es weiter denkbar, dass dem Fußschalter ein Lesegerät zum drahtlosen Auslesen mindestens eines Datenspeichers, vorzugsweise eines aktiven oder passiven RFID, zugeordnet ist. Auf dem Datenspeicher sind personenspezifische Einstellungen oder Konfigurationen oder Autorisierungsdaten für die Benutzung der Vorrichtung abspeicherbar. Der Datenspeicher kann von einer Bedienperson mitgeführt werden; beispielsweise an einer Hose oder am Schuh. Sobald sich die Bedienperson mit dem Datenspeicher dem Fußschalter nähert, wird der Datenträger ausgelesen und bei gegebenenfalls entsprechender Autorisierung der Fußschalter automatisch konfiguriert. So lassen sich die persönlichen, bevorzugten Einstellungen der Bedienperson auf eine schnelle, einfach und zuverlässige Art und Weise auf den Fußschalter übertragen.

Ein Verfahren zur Lösung der eingangs genannten Aufgabe weist die Maßnahmen des Anspruchs 12 auf. Demnach ist es erfindungsgemäß vorgesehen, dass verschiedene Funktionen im Zusammenhang mit dem chirurgischen Gerät durch eine Betätigung eines virtuellen Bedienfeldes ausgeführt werden. Dazu wird insbesondere eine Vorrichtung gemäß mindestens einem der Ansprüche 1 bis 11 verwendet.

Zur Betätigung des virtuellen Bedienfeldes ist es vorgesehen, dass dieses durch einen Fuß oder eine Hand einer Bedienperson bedient wird. Dieses zusätzliche Bedienfeld lässt sich zum einen für die Ausführung nahezu jeder Funktion festlegen und zum anderen auf eine einfache Art und Weise bedienen. Durch die flexible Art und Weise das Bedienfeld auf verschiedene Oberflächen zu projizieren bzw. an verschiedenen Orten zu erstellen, lassen sich die chirurgischen Geräte auf eine besonders einfache und zuverlässige Art und Weise bedienen.

Es kann außerdem vorgesehen sein, dass das virtuelle Bedienfeld bzw. ein Fußschalter automatisch anhand von personenspezifischen Daten, insbesondere Autorisierungsdaten, die auf einem Datenspeicher, vorzugsweise einem aktiven oder passiven RFID, gespeichert sind, konfiguriert wird. Auf dem Datenspeicher werden personenspezifische Einstellungen oder Konfigurationen oder Autorisierungsdaten für die Benutzung der Vorrichtung gespeichert. Der Datenspeicher wird von einer Bedienperson mitgeführt. Sobald sich die Bedienperson mit dem Datenspeicher dem Fußschalter nähert, wird der Datenträger ausgelesen und bei gegebenenfalls entsprechender Autorisierung der Fußschalter automatisch konfiguriert.

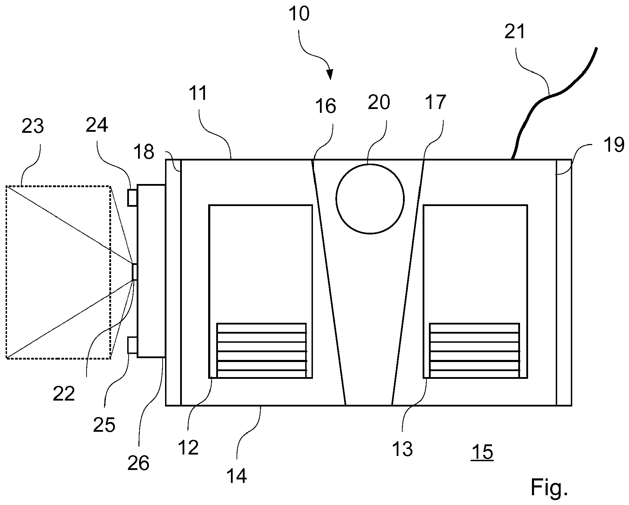

Ein bevorzugtes Ausführungsbeispiel der Erfindung wird nachfolgend anhand der Zeichnung näher erläutert. In der einzigen Figur der Zeichnung ist schematisiert eine Vorrichtung dargestellt mit einem Fußschalter und einem virtuellen Bedienfeld.

Zur Betätigung chirurgischer Geräte werden neben manuellen Betätigungsmitteln auch Betätigungsmittel, die mit den Füßen betätigt werden, eingesetzt. Durch die Verwendung derartiger Fußschalter wird der Aktionsraum einer Bedienperson bzw. eines Operateurs vergrößert. Während der Operateur Funktionen, wie beispielsweise Schneiden von Gewebe, Nähen und dergleichen manuell durchführt, können andere Funktionen im Zusammenhang mit den chirurgischen Geräten, wie beispielsweise die Steuerung der Beleuchtung oder die Aufnahme von Fotos oder Videos durch die Betätigung eines Fußschalters ausgeführt werden. Dabei befinden sich die Fußschalter in der Regel auf einem Fußboden des Behandlungs- bzw. Operationsraumes.

In der Figur ist eine Vorrichtung 10 dargestellt mit einem Fußschalter 11. Der in der Figur dargestellte Fußschalter 11 weist zwei Pedale bzw. Fußpedale 12, 13 auf. Es kann jedoch auch vorgesehen sein, dass der Fußschalter 11 nur ein Pedal oder mehr als zwei Pedale aufweist. In der Regel lassen sich durch diese, ggf. farblich markierten Fußpedale 12, 13 verschiedene Funktionen im Zusammenhang mit chirurgischen Geräten ausführen. Dabei kann es sich bei den Fußpedalen 12, 13 um Taster handeln, welche durch leichte Druckausübung durch den Fuß bzw. eine Fußspitze der Bedienperson eine entsprechende Funktion initiieren.

Die Fußpedale 12, 13 sind für eine einfache Verwendung auf einem Rahmen oder Gehäuse 14 derart angeordnet, dass sie wenigstens nahezu ebenerdig sind. D. h., wenn das Gehäuse 14 des Fußschalters 11 auf einem Fußboden 15 abgestellt wird, sind die Pedale 12, 13 durch leichtes Anwinkeln des Fußes der Bedienperson erreichbar.

Damit der Operateur während der Behandlung bzw. während der Operation nicht ausversehen von einem der beiden Pedale 12, 13 auf das andere Pedal 12, 13 rutscht, bzw. die beiden Fußpedale 12, 13 gleichzeitig betätigt, sind diese jeweils durch einen Steg 16, 17 voneinander getrennt. Die Stege 16, 17 können Bestandteil des Gehäuses sein und ragen in einer Höhe über die Fußpedale 12, 13 hinaus. Ähnliche Stege 18, 19 können auf der gegenüberliegenden Seite der Stege 16, 17 relativ zu den Fußpedalen 12, 13 angeordnet sein, die dem Operateur unter anderem als Orientierungshilfe für die Betätigung der Pedalen 12, 13 dienen können. Für den Fall, dass mehrere Fußschalter 11 nebeneinander auf dem Fußboden 15 angeordnet sind, können die Stege 18, 19 auch verhindern, dass der Operateur bei der Betätigung eines Fußpedals 12, 13 auf die Pedale eines benachbarten Fußschalters 11 rutscht bzw. versehentlich Pedale zweier benachbarter Fußschalter betätigt.

Bei dem in der Figur dargestellten Ausführungsbeispiels eines Fußschalters 11 ist zwischen den beiden Fußpedalen 12, 13 bzw. zwischen den beiden Stegen 16, 17 ein weiteres Betätigungsmittel in Form eines Knopfes 20 angeordnet. Auch dieser Knopf 20 ist durch die beiden Stege 16, 17 von den beiden Fußpedalen 12, 13 getrennt, sodass eine versehentliche Betätigung des Knopfes 20 zusammen mit einem der Fußpedalen 12, 13 nahezu ausgeschlossen werden kann. Über diesen Knopf 20 lassen sich weitere Funktionen im Zusammenhang mit dem chirurgischen Gerät, mit dem der Fußschalter 11 verbunden ist, ausführen. Sowohl die Funktion, die sich durch Betätigung des Knopfes 20 initiieren lässt, als auch die Funktionen die durch die Fußpedale 12, 13 initiierbar sind, können situationsbedingt bzw. den momentanen Anforderungen entsprechend mit verschiedenen Funktionen belegt werden. So ist es denkbar, dass für jede Behandlung bzw. jede Operation die Funktionen der Fußpedale 12, 13 und des Knopfes 20 neu belegt werden. Darüber hinaus ist es denkbar, dass bestimmte Funktionen nur durch eine kombinierte Betätigung des Knopfes 20 mit einem oder beiden der Fußpedalen 12, 13 ausführbar ist. So könnte es vorgesehen sein, dass eine bestimmte Funktion durch das Fußpedal 12 erst initiierbar ist, wenn zuvor der Knopf 20 kurz oder für eine bestimmte Dauer betätigt wurde. Für die Versorgung mit elektrischer Energie kann es vorgesehen sein, dass der Fußschalter 11 durch ein Kabel 21 mit einer Energieversorgung oder mit dem entsprechenden chirurgischen Gerät verbunden ist. Alternativ oder zusätzlich kann es außerdem denkbar sein, dass der Fußschalter 11 durch Batterien bzw. Akkus mit elektrischer Energie versorgt wird.

Neben der Energieversorgung kann das Kabel 21 auch dazu dienen, Informationen zwischen dem Fußschalter 11 und beispielsweise einem chirurgischen Gerät oder einer Steuereinheit auszutauschen. Neben der kabelgebundenen Kommunikation zwischen dem Fußschalter 11 und einer weiteren Einrichtung kann es außerdem vorgesehen sein, dass die Übertragung von Informationen kabellos, sprich über Bluetooth, WLAN, Funk oder dergleichen erfolgen kann. Die kabellose Kommunikation sowie die Versorgung der Vorrichtung 10 mit elektrischer Energie durch Batterien hat den Vorteil, dass keine weiteren Kabel nötig sind, die insbesondere in Anbetracht des begrenzten Raumes auf dem Fußboden 15 des Behandlungsraumes störend sind.

Die in der Figur dargestellte Vorrichtung 10 weist einen an dem Fußschalter 11 angeordneten Projektor 22 auf. Dieser Projektor 22, der beispielsweise als Laserprojektor ausgebildet sein kann, erzeugt auf dem Fußboden 15 ein virtuelles Bedienfeld 23. Dieses virtuelle Bedienfeld 23 kann als zusätzliches Bedienfeld für die Ausführung einer weiteren Funktion im Zusammenhang mit dem chirurgischen Gerät verstanden werden. Genau wie die Fußpedalen 12, 13 des Fußschalters 11 kann auch das Bedienfeld 23 mit dem Fuß betätigt werden. So ist es vorgesehen, dass der Projektor 22 beispielsweise ein Bedienfeld 23 auf den Fußboden 15 projiziert zur Regelung der Leuchtstärke eines Beleuchtungsmittels oder zum Ausführen einer bildlichen Aufnahme. Dazu können entsprechende Symbole oder Befehle auf den Fußboden 15 projiziert werden. Je nach gewünschter Funktion oder Bedarf lässt sich der Projektor 22 vorab derart einstellen, dass er eine entsprechende Projektion bzw. ein virtuelles Bedienfeld 23 auf den Fußboden 15 projiziert.

Das virtuelle Bedienfeld 23 wird von mindestens einem Sensor 24 erfasst. Bei diesem Sensor 24 kann es sich beispielsweise um einen CCD-Chip oder eine Kamera handeln. Sobald sich das virtuelle Bedienfeld 23 verändert bzw. verdunkelt, kann dies durch den Sensor 24 als eine Betätigung des Bedienfeldes 23 durch den Fuß des Operateurs erkannt werden. Diese wahrgenommene Betätigung des virtuellen Bedienfeldes 23 wird sodann von dem Sensor 24 kabellos oder kabelgebunden an eine entsprechende Steuereinrichtung bzw. das entsprechende chirurgische Gerät weitergegeben und die entsprechende Funktion initiiert. Durch einen zweiten Sensor 25, der ähnlich ausgebildet ist wie der Sensor 24, kann die Betätigung des virtuellen Bedienfeldes noch differenziert werden. So ist es denkbar, dass nicht nur Änderungen des virtuellen Bedienfeldes 23 in seiner Erscheinung wahrgenommen werden, sondern auch eine Geste des Fußes, wie beispielsweise ein Entlangführen des Fußes auf dem Fußboden 15. Durch diese Geste bzw. durch dieses Entlangführen des Fußes auf dem Fußboden 15 könnte beispielsweise die Helligkeit eines Leuchtmittels stufenlos verändert werden.

Bei dem in der Figur dargestellten Ausführungsbeispiel der Vorrichtung 10 sind der Projektor 22 und die Sensoren 24, 25 über einen gehäuseartigen Träger 26 mit dem Fußschalter 11 gekoppelt. Genauso kann es vorgesehen sein, dass der Projektor 22 sowie die Sensoren 24, 25 direkt in das Gehäuse 14 des Fußschalters 11 integriert sind.

Ein Ausführungsbeispiel der Erfindung kann es vorsehen, dass eine Vielzahl derartiger Fußschalter 11 mit Projektoren 22 und Sensoren 24, 25 bzw. eine Vielzahl derartiger Träger 26 mit Projektoren 22 und Sensoren 24, 25 auf dem Fußboden 15 platziert werden, um verschiedene Funktionen im Zusammenhang mit verschiedenen chirurgischen Geräten auszuführen.

Auch eine funktionale Kombination der Fußpedale 12, 13 und/oder des Knopfes 20 mit dem virtuellen Bedienfeld 23 ist denkbar. Genau wie zuvor für den Knopf 20 dargestellt, ist es auch denkbar, dass sich Funktionen des virtuellen Bedienfeldes 23 nur ausführen lassen, wenn zuvor der Knopf 20 und/oder eines der Fußpedale 12, 13 betätigt wurde. Auf diese Weise, die Betätigung der verschiedenen Eingabemittel zu kombinieren, lassen sich eine Vielzahl verschiedener Funktionen im Zusammenhang mit einem chirurgischen Gerät ausführen, und zwar ohne, dass weitere platzintensive Fußschalter auf dem Fußboden 15 platziert werden müssen.

### Bezugszeichenliste

- 10: Vorrichtung
- 11: Fußschalter
- 12: Fußpedal
- 13: Fußpedal
- 14: Gehäuse
- 15: Fußboden
- 16: Steg
- 17: Steg
- 18: Steg
- 19: Steg
- 20: Knopf
- 21: Kabel
- 22: Projektor
- 23: Bedienfeld
- 24: Sensor
- 25: Sensor
- 26: Träger

## Patentansprüche

1. Vorrichtung (10) zur Ausführung verschiedener Funktionen im Zusammenhang mit chirurgischen Geräten wie beispielsweise einem Endoskop, einem Resektoskop, einem HF-Resektoskop, einem Kamerasystem, einem HF- oder Ultraschallgenerator, einer Spülpumpe, einer Lichtquellen, einer Wiederaufbereitungsmaschinen oder dergleichen, wobei die Funktionen durch Betätigung eines mindestens ein Fußpedal (12, 13) aufweisenden Fußschalters (11) an der Vorrichtung (10) ausführbar sind, **dadurch gekennzeichnet, dass** der Fußschalter (11) mindestens einen Projektor (22) aufweist zur Erzeugung mindestens eines virtuellen Bedienfeldes (23) und mindestens einen Sensor (24, 25) zur Erfassung des virtuellen Bedienfeldes (23).

2. Vorrichtung (10) nach Anspruch 1, **dadurch gekennzeichnet, dass** der den mindestens einen Projektor (22) und den mindestens einen Sensor (24, 25) aufweisende Fußschalter (11) mit mindestens einem chirurgischen Gerät, insbesondere einem Endoskop, einem Resektoskop, einem HF-Resektoskop, einem Kamerasystem, einem HF- oder Ultraschallgenerator, einer Spülpumpe, einer Lichtquelle, einer Wiederaufbereitungsmaschinen oder dergleichen, über ein Kabel (21) oder kabellos koppelbar ist.

3. Vorrichtung (10) nach Anspruch 1 oder 2, **dadurch gekennzeichnet, dass** das virtuelle Bedienfeld (23) für die Ausführung verschiedener Funktionen im Zusammenhang mit den chirurgischen Geräten konfigurierbar, insbesondere programmierbar, ist.

4. Vorrichtung (10) nach einem der vorherigen Ansprüche, **dadurch gekennzeichnet, dass** über den mindestens einen Sensor (24, 25) eine Betätigung des virtuellen Bedienfeldes (12) durch eine Bedienperson, insbesondere durch einen Fuß oder eine Geste einer Bedienperson, feststellbar ist und durch diese Betätigung die entsprechende Funktion im Zusammenhang mit dem an den Fußschalter (11) gekoppelten chirurgischen Gerät initiierbar ist.

5. Vorrichtung (10) nach einem der vorherigen Ansprüche, **dadurch gekennzeichnet, dass** der mindestens eine Projektor (22) und der mindestens eine Sensor (24, 25) an den mindestens ein Fußpedal (12, 13) aufweisenden Fußschalter (11) anbaubar sind.

6. Vorrichtung (10) nach einem der vorherigen Ansprüche, **dadurch gekennzeichnet, dass** zur kabellosen Übertragung von Informationen zwischen dem Sensor (24, 25) und einem chirurgischen Gerät selbigem ein Receiver zum Senden und/oder Empfangen der Informationen zuordbar ist.

7. Vorrichtung (10) nach einem der vorherigen Ansprüche, **dadurch gekennzeichnet, dass** es sich bei dem Projektor (22) um einen Laserprojektor handelt.

8. Vorrichtung (10) nach einem der vorherigen Ansprüche, **dadurch gekennzeichnet, dass** der mindestens eine Projektor (22) und der mindestens eine Sensor (24, 25) eine eigene Energiequelle, vorzugsweise eine Batterie oder einen Akku aufweisen, oder an die Energieversorgung des Fußschalters (11) gekoppelt sind.

9. Vorrichtung (10) nach einem der vorherigen Ansprüche, **dadurch gekennzeichnet, dass** über das virtuelle Bedienfeld (23) Funktionen ohne Sicherheits- und/oder Gesundheitsrisiken auszuführen sind, wie beispielsweise eine Aufnahme eines Screen-Shots oder dergleichen.

10. Vorrichtung (10) nach einem der vorherigen Ansprüche, **dadurch gekennzeichnet, dass** eine Funktion über das virtuelle Bedienfeld (23) nur nach Betätigung eines Fußpedals (12, 13) des Fußschalters (11) oder eines anderen Bedienelementes ausführbar ist.

11. Vorrichtung (10) nach einem der vorherigen Ansprüche, **dadurch gekennzeichnet, dass** der Fußschalter (11) ein Lesegerät zum drahtlosen Auslesen mindestens eines Datenspeichers, vorzugsweise eines aktiven oder passiven RFID, aufweist, wobei auf dem Datenspeicher personenspezifische Einstellungen oder Konfigurationen oder Autorisierungsdaten für die Benutzung der Vorrichtung (10) speicherbar sind.

12. Verfahren zur Betätigung eines chirurgischen Gerätes wie beispielsweise einem Endoskop, einem Resektoskop, einem HF-Resektoskop, einem Kamerasystem, einem HF- oder Ultraschallgenerator, einer Spülpumpe, einer Lichtquellen, einer Wiederaufbereitungsmaschinen oder dergleichen, **dadurch gekennzeichnet, dass** verschiedene Funktionen im Zusammenhang mit dem chirurgischen Gerät durch eine Betätigung eines virtuellen Bedienfeldes (23) ausgeführt werden, wobei die Funktionen durch Betätigung eines Fußschalters (11) an einer Vorrichtung (10) nach Anspruch 1 ausgeführt werden, und der Fußschalter (11) mindestens einen Projektor (22) aufweist, mit dem das virtuelle Bedienfeld (23) erzeugt wird und das virtuelle Bedienfeld (23) durch mindestens einen Sensor (24, 25) erfasst wird.

13. Verfahren zur Betätigung eines chirurgischen Gerätes nach Anspruch 12, **dadurch gekennzeichnet, dass** das virtuelle Bedienfeld (23) durch einen Fuß, durch eine Hand oder durch eine Geste einer Bedienperson bedient wird.

14. Verfahren zur Betätigung eines chirurgischen Gerätes nach Anspruch 12 oder 13, **dadurch gekennzeichnet, dass** das virtuelle Bedienfeld (23) bzw. ein Fußschalter (11) automatisch konfiguriert wird anhand von personenspezifischen Daten, insbesondere Autorisierungsdaten, die auf einem Datenspeicher, vorzugsweise einem aktiven oder passiven RFID, gespeichert sind und drahtlos ausgelesen werden.

## Claims

1. A device (10) for carrying out various functions in connection with surgical instruments, for example an endoscope, a resectoscope, an HF resectoscope, a camera system, an HF generator or ultrasound generator, a flushing pump, a light source, a reprocessing machine, or the like, wherein the functions can be carried out by actuating a foot switch (11) including at least one foot pedal (12, 13) on the device (10), **characterized in that** the foot switch (11) includes at least one projector (22) for generating at least one virtual control panel (23) and at least one sensor (24, 25) for acquiring the virtual control panel (23).

2. The device (10) as claimed in claim 1, **characterized in that** the foot switch (11) including the at least one projector (22) and the at least one sensor (24, 25) can be coupled to at least one surgical instrument, in particular an endoscope, a resectoscope, an HF resectoscope, a camera system, an HF generator or ultrasound generator, a flushing pump, a light source, a reprocessing machine or the like, via a cable (21) or wirelessly.

3. The device (10) as claimed in claim 1 or 2, **characterized in that** the virtual control panel (23) is configurable, in particular programmable, for carrying out various functions in connection with the surgical instruments.

4. The device (10) as claimed in any one of the preceding claims, **characterized in that** an actuation of the virtual control panel (12) by an operator, in particular by a foot or a gesture of an operator, is establishable via the at least one sensor (24, 25) and the corresponding function can be initiated in connection with the surgical instrument coupled to the foot switch (11) by this actuation.

5. The device (10) as claimed in any one of the preceding claims, **characterized in that** the at least one projector (22) and the at least one sensor (24, 25) can be added onto the foot switch (11) including at least one foot pedal (12, 13).

6. The device (10) as claimed in any one of the preceding claims, **characterized in that** a receiver for transmitting and/or receiving the items of information can be assigned for wireless transmission of items of information between the sensor (24, 25) and a surgical instrument thereof.

7. The device (10) as claimed in any one of the preceding claims, **characterized in that** the projector (22) is a laser projector.

8. The device (10) as claimed in any one of the preceding claims, **characterized in that** the at least one projector (22) and the at least one sensor (24, 25) include a separate energy source, preferably a battery or a rechargeable battery, or are coupled to the energy supply of the foot switch (11).

9. The device (10) as claimed in any one of the preceding claims, **characterized in that** functions without safety and/or health risks are to be carried out via the virtual control panel (23), for example taking a screenshot or the like.

10. The device (10) as claimed in any one of the preceding claims, **characterized in that** a function can be carried out via the virtual control panel (23) only after actuation of a foot pedal (12, 13) of the foot switch (11) or of another operating element.

11. The device (10) as claimed in any one of the preceding claims, **characterized in that** the foot switch (11) includes a read device for wirelessly reading out at least one data memory, preferably an active or passive RFID, wherein person-specific settings or configurations or authorization data for the use of the device (10) are storable on the data memory.

12. A method for actuating a surgical instrument, for example an endoscope, a resectoscope, an HF resectoscope, a camera system, an HF generator or ultrasound generator, a flushing pump, a light source, a reprocessing machine or the like, **characterized in that** various functions are carried out in connection with the surgical instrument by an actuation of a virtual control panel (23), wherein the functions are performed by actuating a foot switch (11) on a device (10) as claimed in claim 1, and the foot switch (11) includes at least one projector (22) with which the virtual control panel (23) is generated and the virtual control panel (23) is acquired by at least one sensor (24, 25).

13. The method for actuating a surgical instrument as claimed in claim 12, **characterized in that** the virtual control panel (23) is operated by a foot, by a hand, or by a gesture of an operator.

14. The method for actuating a surgical instrument as claimed in claim 12 or 13, **characterized in that** the virtual control panel (23) or a foot switch (11) is automatically configured on the basis of person-specific data, in particular authorization data, which are stored on a data memory, preferably an active or passive RFID, and are read out wirelessly.

## Revendications

1. Dispositif (10) de réalisation de diverses fonctions en relation avec des appareils chirurgicaux tels qu'un endoscope, un résectoscope, un résectoscope HF, un système à caméra, un générateur HF ou ultrasons, une pompe de rinçage, une source de lumière, une machine de retraitement ou analogues, les fonctions pouvant être réalisées sur le dispositif (10) par actionnement d'un commutateur de pied (11) comportant au moins une pédale (12, 13), **caractérisé en ce que** le commutateur de pied (11) comporte au moins un projecteur (22) destiné à générer au moins un panneau de commande virtuel (23) et au moins un capteur (24, 25) destiné à détecter le panneau de commande virtuel (23) .

2. Dispositif (10) selon la revendication 1, **caractérisé en ce que** le commutateur de pied (11) comportant l'au moins un projecteur (22) et l'au moins un capteur (24, 25) peut être couplé à au moins un appareil chirurgical, notamment un endoscope, un résectoscope, un résectoscope HF, un système à caméra, un générateur HF ou à ultrasons, une pompe de rinçage, une source de lumière, une machine de retraitement ou similaire, par le biais d'un câble (21) ou sans câble.

3. Dispositif (10) selon la revendication 1 ou 2, **caractérisé en ce que** le panneau de commande virtuel (23) est configurable, notamment programmable, pour réaliser diverses fonctions en relation avec les appareils chirurgicaux.

4. Dispositif (10) selon l'une des revendications précédentes, **caractérisé en ce qu'**un actionnement du panneau de commande virtuel (12) par un opérateur, notamment par un pied ou un geste d'un opérateur, peut être déterminé par le biais d'au moins un capteur (24, 25) et cet actionnement permet de déclencher la fonction correspondante en liaison avec l'appareil chirurgical couplé au commutateur de pied (11) .

5. Dispositif (10) selon l'une des revendications précédentes, **caractérisé en ce que** l'au moins un projecteur (22) et l'au moins un capteur (24, 25) peuvent être fixés au commutateur de pied (11) comportant l'au moins une pédale (12, 13).

6. Dispositif (10) selon l'une des revendications précédentes, **caractérisé en ce qu'**un récepteur destiné à envoyer et/ou recevoir les informations peut être associé à la transmission sans fil d'informations entre le capteur (24, 25) et ce même appareil chirurgical.

7. Dispositif (10) selon l'une des revendications précédentes, **caractérisé en ce que** le projecteur (22) est un projecteur laser.

8. Dispositif (10) selon l'une des revendications précédentes, **caractérisé en ce que** l'au moins un projecteur (22) et l'au moins un capteur (24, 25) disposent de leur propre source d'énergie, de préférence une batterie ou un accumulateur, ou sont couplés à l'alimentation en énergie du commutateur de pied (11).

9. Dispositif (10) selon l'une des revendications précédentes, **caractérisé en ce que** des fonctions peuvent être réalisées par le biais du panneau de commande virtuel (23) sans risque pour la sécurité et/ou la santé, comme par exemple effectuer une capture d'écran ou analogue.

10. Dispositif (10) selon l'une des revendications précédentes, **caractérisé en ce qu'**une fonction peut être réalisée par le biais du panneau de commande virtuel (23) seulement après actionnement d'une pédale (12, 13) du commutateur de pied (11) ou d'un autre élément de commande.

11. Dispositif (10) selon l'une des revendications précédentes, **caractérisé en ce que** le commutateur de pied (11) comporte un appareil de lecture destiné à lire sans fil au moins une mémoire de données, de préférence une RFID active ou passive, des réglages ou configurations ou données d'autorisation spécifiques à une personne et destinés à l'utilisation du dispositif (10) pouvant être mémorisés dans la mémoire de données.

12. Procédé de fonctionnement d'un appareil chirurgical tel qu'un endoscope, un résectoscope, un résectoscope HF, un système à caméra, un générateur HF ou à ultrasons, une pompe de rinçage, une source de lumière, une machine de retraitement ou analogue, **caractérisé en ce que** diverses fonctions en relation avec l'appareil chirurgical peuvent être réalisées par actionnement d'un panneau de commande virtuel (23), les fonctions étant réalisées par actionnement d'un commutateur de pied (11) sur un dispositif (10) selon la revendication 1, et le commutateur de pied (11) comportant au moins un projecteur (22) avec lequel le panneau de commande virtuel (23) est généré et le panneau de commande virtuel (23) est détecté par au moins un capteur (24, 25).

13. Procédé d'actionnement d'un appareil chirurgical selon la revendication 12, **caractérisé en ce que** le panneau de commande virtuel (23) est actionné par un pied, par une main ou par un geste d'un opérateur.

14. Procédé d'actionnement d'un appareil chirurgical selon la revendication 12 ou 13, **caractérisé en ce que** le panneau de commande virtuel (23) ou un commutateur de pied (11) est automatiquement configuré sur la base de données spécifiques à une personne, en particulier de données d'autorisation, qui sont mémorisées dans une mémoire de données, de préférence RFID active ou passive, et lues sans fil.
